# EUROPEAN PATENT SPECIFICATION

(11) **EP 2 244 823 B1**
(45) Date of publication and mention of the grant of the patent: **03.08.2011**
(21) Application number: 08852296.6
(22) Date of filing: 19.11.2008
(51) Int. Cl.: B01J 8/18, B01J 8/26, C07C 1/20, C07C 11/02

(54) **PROCESS FOR THE PREPARATION OF AN OLEFINIC PRODUCT**
VERFAHREN ZUR HERSTELLUNG EINES OLEFINISCHEN PRODUKTS
PROCÉDÉ DE PRÉPARATION D'UN PRODUIT OLÉFINIQUE

(30) Priority: 19.11.2007 EP 07121003
(43) Date of publication of application: 03.11.2010
(73) Proprietor: Shell Internationale Research Maatschappij B.V., 2596 HR The Hague (NL)
(72) Inventor: CHEWTER, Leslie, Andrew, NL-1031 CM Amsterdam (NL); VAN WESTRENEN, Jeroen, NL-1031 CM Amsterdam (NL); WINTER, Ferry, NL-1031 CM Amsterdam (NL)
(74) Representative: Matthezing, Robert Maarten
(86) International application number: PCT/EP2008/065812
(87) International publication number: WO 2009/065848

(56) References cited:
- EP-A- 0 485 145
- EP-A- 0 489 497
- WO-A-01/62689
- WO-A-03/020667
- DE-A1- 10 027 159

## Description

This invention relates to a process for the preparation of an olefinic product, in particular including lower olefins such as ethylene and/or propylene. More in particular this invention relates to a process for the conversion of oxygenates into olefins.

Processes for the preparation of olefins from oxygenates are known in the art.

US 6 797 851 describes a process for making ethylene and propylene from an oxygenate feed. The process is conducted in two stages using two different oxygenate conversion catalysts, wherein in the first stage oxygenates are converted to a light olefin stream, and wherein in the second stage C4+ olefins produced in the first stage are converted to additional ethylene and propylene. The catalyst used for the first stage is a ZSM-5 containing oxygenate conversion catalyst. The second stage catalyst is a 10-ring zeolite, and ZSM-22, ZSM-23, ZSM-35, ZSM-48 are mentioned. ZSM-35 is preferred. Various embodiments of reaction systems with first and second stage catalyst in separate reaction zones are discussed. Without disclosing an embodiment, it is generally mentioned that the two catalysts can be mixed.

In the known process, significant amounts of aromatics are produced. Most aromatics are produced in the first stage by the conversion of oxygenate over ZSM-5 zeolite, and once formed, aromatics are unlikely to be converted into olefins in the second stage.

In Example 2, obtained with ZSM-5 and ZSM-35 the final product after the second stage contains 11 wt% of aromatics.

A positive influence of aromatics in the conversion of methanol or dimethylether to a product containing C2 to C4 olefins is disclosed in US patent specification No. 6 046 372. In the known process, the conversion takes place by contacting a feed containing methanol and/or dimethylether in the presence of an aromatic compound with a catalyst comprising a porous crystalline material, in particular zeolite ZSM-5. In the examples, which were all conducted with ZSM-5, it had been found that the addition of an aromatic compound to the methanol and/or dimethylether feed increased the selectivity of the conversion reaction towards ethylene.

It is desired to provide a new process that allows to maximise production of light olefins, in particular with a high selectivity for ethylene from an oxygenate feedstock.

In accordance with the present invention there is provided a process for the preparation of an olefinic product, which process comprises the steps of
a) reacting an oxygenate feedstock comprising oxygenate species having an oxygen-bonded methyl group, preferably methanol and/or dimethylether, and an olefinic co-feed, in the presence of an oxygenate conversion catalyst comprising at least 50 wt%, based on total molecular sieve in the oxygenate conversion catalyst, of a molecular sieve having one-dimensional 10-membered ring channels, to prepare an olefinic reaction effluent, wherein the olefinic co-feed comprises less than 10 wt% of C5+ hydrocarbon species;
b) fractionating the olefinic reaction effluent to obtain at least a light olefinic fraction comprising ethylene, and a heavier olefinic fraction comprising C4 olefins and less than 10 wt% of C5+ hydrocarbon species;
c) recycling at least part of the heavier olefinic fraction obtained in step b) as recycle stream to step a), to form at least part of the olefinic co-feed; and
d) withdrawing at least part of the light olefinic fraction as olefinic product.

The process of the invention allows maximising of light olefin production, such as ethylene and/or propylene production, from an oxygenate feedstock comprising e.g. methanol and/or dimethylether. It has been found that an oxygenate conversion catalyst including a majority of a molecular sieve having one-dimensional 10-membered ring channels is particularly effective for this purpose, in particular in the case wherein the reaction mixture comprises an olefinic co-feed in addition to the oxygenate.

The expression 'molecular sieve' is used in the description and claims for a material containing small regular pores and/or channels and exhibiting catalytic activity in the conversion of oxygenate to olefin. A molecular sieve having one-dimensional 10-membered ring channels is understood to be a molecular sieve having only 10-membered ring channels in one direction, which are not intersected by other 8, 10 or 12-membered ring channels from another direction. The molecular sieve having one-dimensional 10-membered ring channels and/or the more-dimensional molecular sieve can in particular be a zeolite. A zeolite is understood to be an aluminosilicate molecular sieve. Where reference is made in the description and in the claims to a molecular sieve, this can in particular be a zeolite.

The olefinic co-feed comprises less than 10 wt% of C5+ olefins, in particular less than 10 wt% of C5+ olefins and paraffins, more in particular less than 10 wt% of C5+ hydrocarbon species. C5+ denotes hydrocarbons with 5 or more carbon atoms.

Without wishing to be bound by a particular hypothesis or theory, applicant presently believes that a molecular sieve having one-dimensional 10-membered ring channels, such as MTT type molecular sieves and/or TON type molecular sieves, are particular effective in converting oxygenate species having an oxygen-bonded methyl group, such as methanol and/or dimethylether, together with a C4 olefin comprising co-feed. In particular, applicant believes that in such molecular sieves alkylation of olefins and subsequent cracking occurs in a favourable fashion leading to low production of by-products such as aromatics, saturates, C5+ hydrocarbon species, methane, carbon oxides, and rather to a high yield of light olefins with a significant portion of valuable ethylene. More in particular it is currently believed that an optimum reaction pathway includes alkylation of a C4 olefin by the methyl group of the oxygenate species, followed by cracking of the resulting C5 olefin into ethylene and propylene molecules. According to the invention, the amount of C5+ olefins in the feed to the reactor should be limited, since alkylation of such higher olefins followed by cracking results in lower selectivity towards ethylene.

Further according to the invention, the olefinic co-feed is at least partially obtained by recycling a suitable fraction comprising C4 olefin.

In one embodiment at least 70 wt% of the olefinic co-feed in step a), during normal operation, is formed by the recycle stream of step c), preferably at least 90 wt%, more preferably at least 99 wt%. Most preferably the olefinic co-feed is during normal operation formed by the recycle stream, so that the process converts oxygenate feedstock to predominantly light olefins without the need for an external olefins stream. During normal operation means for example in the course of a continuous operation of the process, for at least 70% of the time on stream. The olefinic co-feed may need to be obtained from an external source, such as from a catalytic cracking unit or from a naphtha cracker, during start-up of the process, when the reaction effluent comprises no or insufficient heavier fraction including C4 olefins.

In a preferred embodiment the heavier olefinic fraction comprises at least 50 wt% of C4 olefins, and at least a total of 70 wt% of C4 hydrocarbon species. Suitably the heavier olefinic fraction is at least partially obtained as a C4 fraction of the olefinic reaction effluent. The C4 fraction contains C4 olefin(s), but can also contain a significant amount of other C4 hydrocarbon species, in particular C4 paraffins, because it is difficult to economically separate C4 olefins and paraffins, such as by distillation. The heavier olefinic fraction can in particular contain at least a total of 90 wt% of C4 hydrocarbon species. In a preferred embodiment, the olefinic co-feed, comprises less than 5 wt% of C5+ olefins, preferably less than 2 wt% of C5+ olefins, even more preferably less than 1 wt% of C5+ olefins, and likewise the recycle stream. The recycle stream can comprises less than 5 wt% of C5+ olefins, preferably less than 2 wt% of C5+ olefins, based on total hydrocarbons in the recycle stream. In another preferred embodiment, the olefinic co-feed, comprises less than 5 wt% of C5+ hydrocarbon species, preferably less than 2 wt% of C5+ hydrocarbon species even more preferably less than 1 wt% of C5+ hydrocarbon species, and likewise the recycle stream.

The heavier olefinic fraction can also comprise propylene. This may be preferred when a particularly high production of ethylene is desired, so that part or all of the propylene produced is recycled together with C4 olefins.

Suitably the olefinic reaction effluent comprises less than 10 wt%, preferably less than 5 wt%, more preferably less than 1 wt%, of C6-C8 aromatics. Producing low amounts of aromatics is desired since any production of aromatics consumes oxygenate which is therefore not converted to lower olefins.

Suitably the reaction in step a) is conducted at a temperature of more than 450 °C, preferably at a temperature of 460 °C or higher, in particular 480 °C or higher, more preferably at a temperature of 490 °C or higher. At higher temperatures a higher ethylene selectivity is observed.

In a preferred embodiment the molecular sieve having one-dimensional 10-membered ring channels ("one-dimensional molecular sieve") comprises at least one of a molecular sieve of the MTT-type and/or of the TON-type. Examples are ZSM-23 for MTT, and ZSM-22 for TON.

Preferably the one-dimensional molecular sieve has a silica to alumina ratio (SAR) in the range from 1 to 500, preferably in the range from 10 to 200. The SAR is defined as the molar ratio of SiO₂/Al₂O₃ corresponding to the composition of the molecular sieve.

For ZSM-22, a SAR in the range of 40-150 is preferred, in particular in the range of 70-120. Good performance in terms of activity and selectivity has been observed with a SAR of about 100.

For ZSM-23, an SAR in the range of 20-120 is preferred, in particular in the range of 30-80. Good performance in terms of activity and selectivity has been observed with a SAR of about 50.

In one embodiment the oxygenate conversion catalyst can comprise more than 50 wt%, at least 65 wt%, based on total molecular sieve in the oxygenate conversion catalyst, of the one-dimensional molecular sieve having 10-membered ring channels. The presence of a majority of such molecular sieve strongly determines the predominant reaction pathway.

In a special embodiment the oxygenate conversion catalyst comprises at least 1 wt%, based on total molecular sieve in the oxygenate conversion catalyst, of a further molecular sieve having more-dimensional channels, in particular at least 5 wt%, more in particular at least 8 wt%. The presence of a minority portion of a more-dimensional molecular sieve in the oxygenate conversion catalyst was found to improve stability (slower deactivation during extended runs) and hydrothermal stability. Without wishing to be bound by a particular hypothesis or theory, it is presently believed that this is due to the possibility for converting larger molecules by the more-dimensional molecular sieve, that were produced by the one-dimensional molecular sieve, and which would otherwise form coke. The further molecular sieve can for example be a MFI-type molecular sieve such as ZSM-5, or a SAPO-type molecular sieve such as SAPO-34. The weight ratio between the molecular sieve having one-dimensional 10-membered ring channels, and the further molecular sieve having more-dimensional channels can be in the range of from 1:1 to 100:1.

Preferably the further molecular sieve is a MFI-type molecular sieve, in particular zeolite ZSM-5, having a Silica-to-Alumina ratio SAR of at least 60, more preferably at least 80, even more preferably at least 100, yet more preferably at least 150. At higher SAR the percentage of C4 saturates in the C4 totals produced is minimized. In special embodiments the oxygenate conversion catalyst can comprise less than 35 wt% of the further molecular sieve, based on the total molecular sieve in the oxygenate conversion catalyst, in particular less than 20 wt%, more in particular less than 18 wt%, still more in particular less than 15 wt%.

The process of the present invention enables the conversion of the oxygenate feed to olefins over a molecular sieve having one-dimensional 10-membered ring channels which may otherwise be cumbersome. Such is illustrated in table 2B of EP-A 0485145 where it is shown that one-dimensional zeolites having 10-membered ring channels, such as zeolites of the TON-type, are not capable of converting an oxygenate at a reasonable rate in the absence of any olefin. Moreover, the process of the present invention provides for high ethylene yield.

By an olefinic composition or stream, such as an olefinic product, product fraction, fraction, effluent, reaction effluent or the like is understood a composition or stream comprising one or more olefins, unless specifically indicated otherwise. Other species can be present as well. The olefinic composition or stream can comprise one type of olefin or a mixture of olefins.

By an olefin is understood an organic compound containing at least two carbon atoms connected by a double bond.

In particular the olefinic co-feed can contain a mixture of olefins. Apart from olefins, the olefinic co-feed may contain other hydrocarbon compounds, such as for example paraffinic compounds. Preferably the olefinic co-feed comprises an olefinic portion of more than 50 wt%, more preferably more than 60 wt%, still more preferably more than 70 wt%, which olefinic portion consists of olefin(s). The olefinic co-feed can also consist essentially of olefin(s).

Any non-olefinic compounds in the olefinic co-feed are preferably paraffinic compounds. Such paraffinic compounds are preferably present in an amount in the range from 0 to 50 wt%, more preferably in the range from 0 to 40 wt%, still more preferably in the range from 0 to 30 wt%.

The olefin can be a mono-olefin, having one double bond, or a poly-olefin, having two or more double bonds. Preferably olefins present in the olefinic co-feed are mono-olefins. C4 olefins, also referred to as butenes (1-butene, 2-butene, iso-butene, and/or butadiene), in particular C4 mono-olefins, are preferred components in the olefinic co-feed. Preferably the olefinic portion of the olefinic co-feed, and of the recycle stream, comprises at least 90 wt% of C4 olefins, more preferably at least 99 wt%. Butenes as co-feed have been found to be particularly beneficial for high ethylene selectivity.

One particularly suitable recycle stream consists essentially, i.e. for at least 99 wt%, of 1-butene, 2-butene (cis and trans), isobutene, n-butane, isobutene, butadiene. The skilled artisan knows how to obtain such a fractions from the olefinic reaction effluent such as by distillation.

The oxygenate feedstock comprises oxygenate species having an oxygen-bonded methyl group, such as methanol, dimethylether. Preferably the oxygenate feedstock comprises at least 50 wt% of methanol and/or dimethylether, more preferably at least 80 wt%, most preferably at least 90 wt%. The oxygenate feedstock can be obtained from a prereactor, which converts methanol at least partially into dimethylether. In this way, less water is formed in the process of converting oxygenate to olefins, which has advantages for the process design and lowers the severity of hydrothermal conditions the catalyst is exposed to. The oxygenate feedstock can comprise an amount of water, preferably less than 10 wt%, more preferably less than 5 wt%. Preferably the oxygenate feedstock contains essentially no hydrocarbons other than oxygenates, i.e. less than 5 wt%, preferably less than 1 wt%.

In one embodiment, the oxygenate is obtained as a reaction product of synthesis gas. Synthesis gas can for example be generated from fossil fuels, such as from natural gas or oil, or from the gasification of coal. Suitable processes for this purpose are for example discussed in Industrial Organic Chemistry, Klaus Weissermehl and Hans-Jürgen Arpe, 3rd edition, Wiley, 1997, pages 13-28. This book also describes the manufacture of methanol from synthesis gas on pages 28-30.

In another embodiment the oxygenate is obtained from biomaterials, such as through fermentation. For example by a process as described in DE-A-10043644.

The preferred molar ratio of oxygenate in the oxygenate feedstock to olefin in the olefinic co-feed depends on the specific oxygenate used and the number of reactive oxygen-bonded alkyl groups therein. Preferably the molar ratio of oxygenate to olefin in the total feed lies in the range of 10:1 to 1:10, more preferably in the range of 5:1 to 1:5 and still more preferably in the range of 3:1 to 1:3.

In a preferred embodiment wherein the oxygenate comprises only one oxygen-bonded methyl group, such as methanol, the molar ratio preferably lies in the range from 5:1 to 1:5 and more preferably in the range of 2.5:1 to 1:2.5.

In another preferred embodiment wherein the oxygenate comprises two oxygen-bonded methyl groups, such as for example dimethylether, the molar ratio preferably lies in the range from 5:2 to 1:10 and more preferably in the range of 2:1 to 1:4. Most preferably the molar ratio in such a case is in the range of 1.5:1 to 1:3.

Step a) of the process is carried out in presence of a molecular sieve having one-dimensional 10-membered ring channels. These are understood to be molecular sieves having only 10-membered ring channels in one direction which are not intersected by other 8, 10 or 12-membered ring channels from another direction.

Preferably, the molecular sieve is selected from the group of TON-type (for example zeolite ZSM-22), MTT-type (for example zeolite ZSM-23), STF-type (for example SSZ-35), SFF-type (for example SSZ-44), EUO-type (for example ZSM-50), and EU-2-type molecular sieves or mixtures thereof.

MTT-type catalysts are more particularly described in e.g. US-A-4,076,842. For purposes of the present invention, MTT is considered to include its isotypes, e.g., ZSM-23, EU-13, ISI-4 and KZ-1.

TON-type molecular sieves are more particularly described in e.g. US-A-4,556,477. For purposes of the present invention, TON is considered to include its isotypes, e.g., ZSM-22, Theta-1, ISI-1, KZ-2 and NU-10.

EU-2-type molecular sieves are more particularly described in e.g. US-A-4,397,827. For purposes of the present invention, EU-2 is considered to include its isotypes, e.g., ZSM-48.

In a further preferred embodiment a molecular sieve of the MTT-type, such as ZSM-23, and/or a TON-type, such as ZSM-22 is used.

Molecular sieve and zeolite types are for example defined in Ch. Baerlocher and L.B. McCusker, Database of Zeolite Structures: http://www.izastructure.org/databases/, which database was designed and implemented on behalf of the Structure Commission of the International Zeolite Association (IZA-SC), and based on the data of the 4th edition of the Atlas of Zeolite Structure Types (W.M. Meier, D.H. Olson and Ch. Baerlocher).

The process of the invention can be carried out in the presence of only one or more molecular sieves having one-dimensional 10-membered ring channels, in particular of just one type of molecular sieve having one-dimensional 10-membered ring channels.

Preferably, molecular sieves in the hydrogen form are used in the oxygenate conversion catalyst, e.g., HZSM-22, HZSM-23, and HZSM-48, HZSM-5. Preferably at least 50% w/w, more preferably at least 90% w/w, still more preferably at least 95% w/w and most preferably 100% of the total amount of molecular sieve used is in the hydrogen form. When the molecular sieves are prepared in the presence of organic cations the molecular sieve may be activated by heating in an inert or oxidative atmosphere to remove organic cations, for example, by heating at a temperature over 500 °C for 1 hour or more. The zeolite is typically obtained in the sodium or potassium form. The hydrogen form can then be obtained by an ion exchange procedure with ammonium salts followed by another heat treatment, for example in an inert or oxidative atmosphere at a temperature over 500 °C for 1 hour or more. The molecular sieves obtained after ionexchange are also referred to as being in the ammonium form.

The molecular sieve can be used as such or in a formulation, such as in a mixture or combination with a so-called binder material and/or a filler material, and optionally also with an active matrix component. Other components can also be present in the formulation. If one or more molecular sieves are used as such, in particular when no binder, filler, or active matrix material is used, the molecular sieve itself is/are referred to as oxygenate conversion catalyst. In a formulation, the molecular sieve in combination with the other components of the mixture such as binder and/or filler material is/are referred to as oxygenate conversion catalyst.

It is desirable to provide a catalyst having good mechanical or crush strength, because in an industrial environment the catalyst is often subjected to rough handling, which tends to break down the catalyst into powder-like material. The latter causes problems in the processing. Preferably the molecular sieve is therefore incorporated in a binder material. Examples of suitable materials in a formulation include active and inactive materials and synthetic or naturally occurring zeolites as well as inorganic materials such as clays, silica, alumina, silica-alumina, titania, zirconia and aluminosilicate. For present purposes, inactive materials of a low acidity, such as silica, are preferred because they may prevent unwanted side reactions which may take place in case a more acidic material, such as alumina or silica-alumina is used.

The process of the present invention can be carried out in a batch, continuous, semi-batch or semi-continuous manner. Preferably the process of the present invention is carried out in a continuous manner.

If the process is carried out in a continuous manner, the process may be started up by using olefins obtained from an external source for the olefinic co-feed in step a). Such olefins may for example be obtained from a steam cracker, a catalytic cracker, alkane dehydrogenation (e.g. propane or butane dehydrogenation). Further, such olefins can be bought from the market.

In a special embodiment the olefins for such start-up are obtained from a previous process that converted oxygenates, with or without olefinic co-feed, to olefins. Such a previous process may have been located at a different location or it may have been carried out at an earlier point in time.

When a more-dimensional molecular sieve such as ZSM-5 is present in the oxygenate conversion catalyst, even in minority compared to the one-dimensional molecular sieve, start up is possible without an olefinic co-feed from an external source. ZSM-5 for example is able to convert an oxygenate to an olefin-containing product, so that a recycle can be established.

The reactor system used in step a) may be any reactor known to the skilled person and may for example contain a fixed bed, moving bed, fluidized bed, riser reactor and the like. A riser reactor system is preferred, in particular a riser reactor system comprising a plurality of serially arranged riser reactors.

Typically the oxygenate conversion catalyst deactivates in the course of the process. Conventional catalyst regeneration techniques can be employed. The one-dimensional molecular sieve having 10 membered ring channels used in the process of the present invention can have any shape known to the skilled person to be suitable for this purpose, for it can be present in the form of spheres, tablets, rings, extrudates, etc. Extruded catalysts can be applied in various shapes, such as, cylinders and trilobes. If desired, spent oxygenate conversion catalyst can be regenerated and recycled to the process of the invention.

Step a) of the process can be carried out over a wide range of temperatures and pressures. Suitably, however, the oxygenate feed and olefinic co-feed are contacted with the molecular sieve at a temperature in the range from 200 °C to 650 °C. In a further preferred embodiment the temperature is in the range from 250 °C to 600 °C, more preferably in the range from 300 °C to 550 °C, most preferably in the range from 450 °C to 550 °C. Preferably the reaction in step a) is conducted at a temperature of more than 450 °C, preferably at a temperature of 460 °C or higher, more preferably at a temperature of 490 °C or higher. At higher temperatures a higher activity and ethylene selectivity is observed. One-dimensional molecular sieves having 10-membered ring channels can be operated under oxygenate conversion conditions at such high temperatures with acceptable deactivation due to coking, contrary to molecular sieves with smaller pores or channels, such as 8-membered ring channels. Temperatures referred to hereinabove represent reaction temperatures, and it will be understood that a reaction temperature can be an average of temperatures of various feed streams and the catalyst in the reaction zone.

In addition to the oxygenate, and the olefinic co-feed, a diluent may be fed into the reactor system. It is preferred to operate without a diluent, or with a minimum amount of diluent, such as less than 200 wt% of diluent based on the total amount of oxygenate feed, in particular less than 100 wt%, more in particular less than 20 wt%. Any diluent known by the skilled person to be suitable for such purpose can be used. Such diluent can for example be a paraffinic compound or mixture of compounds. Preferably, however, the diluent is an inert gas. The diluent can be argon, nitrogen, and/or steam. Of these, steam is the most preferred diluent. For example, the oxygenate feed and optionally olefinic co-feed can be diluted with steam, for example in the range from 0.01 to 10 kg steam per kg oxygenate feed.

In one embodiment small amounts of water are added to step a) in order to improve the stability of the catalyst by reducing coke formation.

In step b) of the process according to the invention the olefinic reaction effluent of step a) is fractionated. The skilled artisan knows how to separate a mixture of hydrocarbons into various fractions, and how to work up fractions further for desired properties and composition for further use. The separations can be carried out by any method known to the skilled person in the art to be suitable for this purpose, for example by vapour-liquid separation (e.g. flashing), distillation, extraction, membrane separation or a combination of such methods. Preferably the separations are carried out by means of distillation. It is within the skill of the artisan to determine the correct conditions in a fractionation column to arrive at such a separation. He may choose the correct conditions based on, inter alia, fractionation temperature, pressure, trays, reflux and reboiler ratios.

At least a light olefinic fraction comprising ethylene and a heavier olefinic fraction comprising C4 olefins and less than 10 wt% of C5+ hydrocarbon species are obtained. Preferably also a water-rich fraction is obtained. Also a lighter fraction comprising methane, carbon monoxide, and/or carbon dioxide can be obtained, as well as one or more heavy fractions comprising C5+ hydrocarbons. Such heavy fraction can for example be used as gasoline blending component.

In the process also a significant amount of propylene is produced. The propylene can form part of the light olefinic fraction comprising ethene, and which can suitably be further fractionated into various product components. Propylene can also form part of the heavier olefinic fraction comprising C4 olefins. The various fractions an streams referred to herein, in particular the recycle stream, can be obtained by fractionating in various stages, and also by blending streams obtained during the fractionation. Typically, an ethylene and a propylene stream of predetermined purity such as export quality will be obtained from the process, and also a stream rich in C4 comprising C4 olefins and optionally C4 paraffins. It shall be clear that the heavier olefinic fraction comprising C4 olefins, forming the recycle stream in step c), can be composed from quantities of various fractionation streams. So, for example, some amount of a propylene-rich stream can be blended into a C4 olefin-rich stream. In a particular embodiment at least 90 wt% of the heavier olefinic fraction comprising C4 olefins can be the formed by the overhead stream from a debutaniser column receiving the bottom stream from a depropanizer column at their inlet, more in particular at least 99 wt% or substantially all.

Aspects of the invention will be explained in more detail and by way of example, with reference to the drawings, wherein
Figure 1 shows schematically an embodiment of the present invention; and
Figure 2 schematically shows a reaction network; and
Figure 3 shows schematically a further embodiment of the invention.

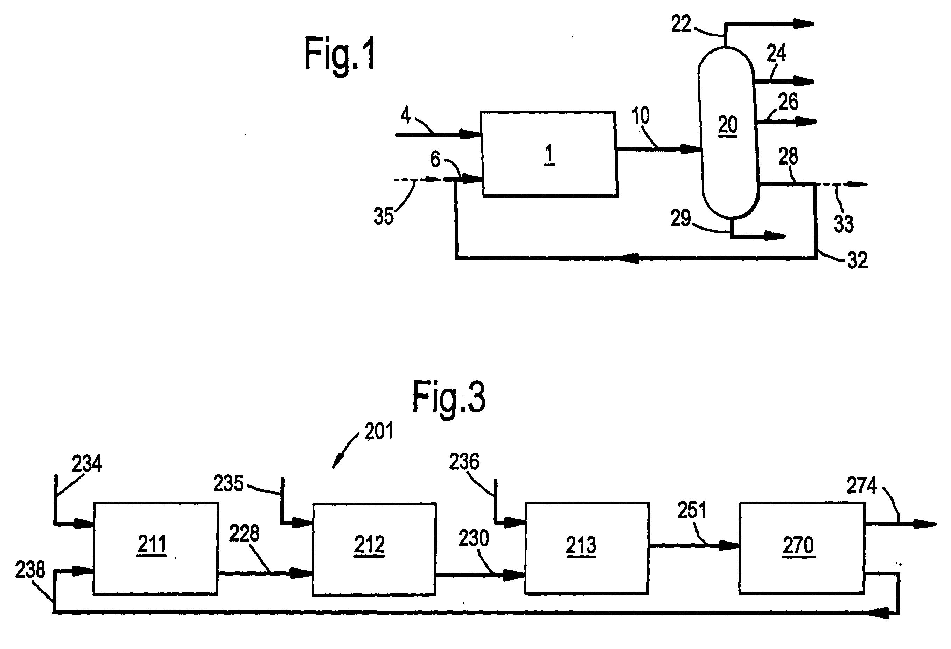

Reference is made to Figure 1. An oxygenate feedstock as specified herein above is fed to a reactor system 1 via line 4. An olefinic co-feed, as specified herein above, is fed to the reactor system as well, via line 6. In the reactor system 1, the oxygenate feedstock and the olefinic co-feed are allowed to react in the presence of an oxygenate conversion catalyst as specified herein above, to prepare an olefinic reaction effluent in line 10.

The olefinic reaction effluent is sent to a fractionation section 20. In this embodiment the fractionation section is shown to produce an ethylene-rich product stream in line 24 as light olefinic fraction, and a C4-olefin-rich stream in line 28, as heavier olefinic fraction specified herein above, and further a lighter stream in overhead line 22 comprising lighter contaminants such as methane and/or carbon oxides, a propylene-rich stream in line 26 and a C5+ hydrocarbon rich stream in line 29.

At least part of the heavier olefinic fraction in line 28 is recycled via line 32 to an inlet of the reactor system 1, to form at least part of the olefinic co-feed. If desired, part of the heavier olefinic fraction can be withdrawn via line 33. If this is merely a small bleed stream, such as less than 10 wt% or in particular less than 5 wt%, substantially all of the heavier olefinic fraction is considered to be recycled. To the recycle stream other components can be blended, such as from the propylene rich stream 26 or from the C5+ hydrocarbon-rich stream 29. The latter can increase yield of lower olefins, but is less desired because it does so at the cost of ethylene selectivity.

In special and not generally preferred embodiments, such as during start-up, part of the olefinic co-feed can be obtained from an external source via line 35. If that is not the case, the overall process converts oxygenate in line 4 to mainly light olefins in lines 24 and 26.

### Examples

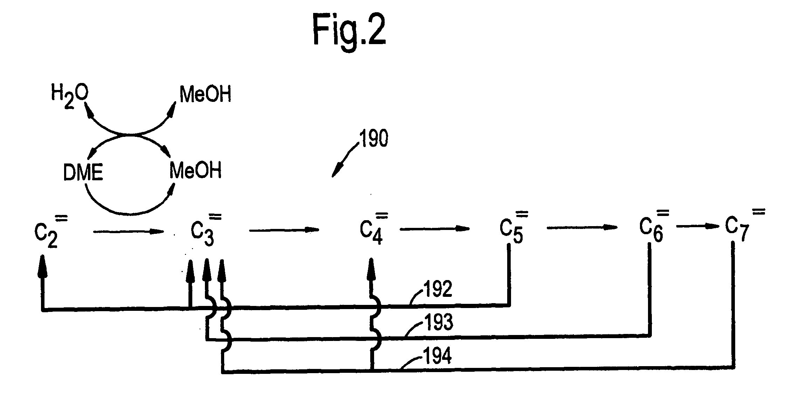

In the examples, also results from model calculations of various reactor systems are presented. These calculations were based on a kinetic reaction network 190 depicted in Figure 2. This reaction network represents the alkylation and cracking of C2-C7 olefins, in the presence of oxygenates comprising dimethylether (DME) and/or methanol (MeOH). The reaction conditions are such that higher than C7 olefins are hardly formed and can be neglected. In the alkylation it is assumed that DME reacts with an olefin, producing methanol, and that 2 methanol molecules are dehydrated to DME. Water is being obtained as a reaction product from alkylation. This cycle is indicated in Figure 2 for the alkylation from ethylene to propylene, and it shall be clear that it also applies to the alkylation steps of C3= and higher olefins. C2-C4 olefins are not cracked under the prevailing operating conditions. Cracking of one C5= molecule results in one C2= and one C3= molecule, cracking of C6= in two C3= molecules, and cracking of C7= in one C3= and one C4= molecule. This is illustrated by the arrows 192, 193, 194.

On the basis of the reaction network 190 a kinetic model was established, and parameters of the model were determined based on experiments of oxygenate (DME and/or MeOH) and various olefin feed conversions over ZSM-23. The kinetic model was then used in various reactor models as discussed below. Aspen Custom Modeller was used in this process, together with proprietary software routines.

The model does not take into account the production or conversion of paraffins. It is known that some paraffins are being formed over an oxygenate conversion catalyst. One of the options of practical interest is the recycle of C4= olefins from the reaction product to the inlet of the reactor system. It is known that separating C4 olefins from C4 paraffins is difficult, and may not be economically attractive. Therefore the recycle stream may contain C4 paraffins (C4,0) together with C4=. Paraffins such as butane can be regarded as inerts at typical oxygenation conditions over the oxygenate conversion catalysts, therefore a certain level of paraffins (butane) will build up. In some Examples below, the role of C4 paraffins has been included. In order to mimic their production in the process, a small amount was included in the oxygenate feed, and a corresponding bleed stream was taken out of the product fraction. The net effect is a level of C4,0 in the process, which is further assumed to be inert.

### Example 1

Model calculations for a reactor system 1 as in Figure 1 were conducted, wherein the reactor system was formed by an isothermal reactor. The catalyst amount assumed in the model was 5 tonnes. 40 wt% of the catalyst was assumed to be zeolite. A uniform temperature of 522 °C and pressure of 1 bar was assumed. In the reactor model oxygenate feedstock (DME, MeOH, some H₂O) was fed via line 4, and C4 olefin was fed via line 6. Products (ethylene, propylene, C5 olefins and water) are withdrawn after separation from C4 olefins in separation system 20. C4= is recycled to the first reactor, and in addition C4 saturates C4,0 are taken into account as discussed above.

The composition of various streams indicated by the reference numerals of Figure 1 are given in Table 1.

**Table 1**

| Stream (kmol/h) | lines 32;6 | line 4 | line 10 |
|---|---|---|---|
| DME | | 2766 | 0.0 |
| MeOH | | 978 | 1 |
| H₂O | | | 3742 |
| C₂⁼ | | | 674 |
| C₃⁼ | | | 1646 |
| C₄⁼ | 897 | | 944 |
| C₄⁰ | 418 | 22 | 440 |
| C₅⁼ | | | 7 |
| C₆⁼ | | | 0 |
| C₇⁼ | | | 0 |
| Total | 1315 | 3766 | 7455 |
| Total (t/h) | 74 | 160 | 234 |

The process according to the invention converts a pure oxygenate feedstock into predominantly ethylene and propylene. The C4 olefins formed are essentially fully recycled. In the model a bleed of 5 mol% was applied for the C4 olefins and saturates C4= and C4,0, in order to prevent build up of C4,0. The main further product (effluent) from the process is water.

### Comparative example 2

A complementary model calculation of the overall reaction but without a C4 olefin recycle was performed. Instead of recycling a fraction of the effluent through line 32 (closed), the model included a C4 feed stream comprising olefins and saturates via line 35,6. The further parameters were kept constant.

The composition of various streams indicated by the reference numerals of Figure 1 are given in Table 2.

**Table 2**

| Stream (kmol/h) | lines 35;6 | line 4 | line 10 |
|---|---|---|---|
| DME | | 2766 | 0 |
| MeOH | | 978 | 1 |
| H₂O | | | 3743 |
| C₂⁼ | | | 669 |
| C₃⁼ | | | 1645 |
| C₄⁼ | 897 | | 949 |
| C₄⁰ | 22 | | 22 |
| C₅⁼ | | | 5 |
| C₆⁼ | | | 0 |
| C₇⁼ | | | 0 |
| Total | 919 | 4663 | 7034 |
| | 52 | 156 | |
| Total(t/h) | | | 210 |

This example illustrates, that the incoming C4 stream is hardly converted in net effect. C4 olefin mainly plays the role of a template for the conversion from oxygenate to light olefins ethylene and propylene.

### Comparative Example 3

A methanol containing feed, in the absence of an olefinic co-feed, was reacted over a MTT zeolite ZSM-23 with a silica-to-alumina ratio of 48. The reactor was heated in argon to the reaction temperature of 500 °C, and a mixture consisting of 8 vol.% methanol in argon was passed over the catalyst at atmospheric pressure at a flow rate of 100 ml/min. Gas hourly space velocity (GHSV) is 60,000, based on total gas flow. Weight hourly space velocities (WHSV) is 6.9 gram methanol/gram catalyst/hr, based on methanol mass flow. The effluent from the reactor was analyzed by mass spectrometry to determine the product composition. The results are shown in Table 3.

**Table 3**

| Catalyst | MTT-type |
|---|---|
| Time on stream, hr | 1 |
| Methanol conversion, % | 75 |
| Methanol conc., vol.% | 2.0 |
| DME conc., vol.% | 3.0 |
| Total olefin conc., vol.% | <0.2 |

As can be seen from the above the process without any olefinic co-feed results in a low conversion to olefins. This confirms the teaching of EP-A 0485145.

### Examples 4a and 4b

In these examples dimethyl ether (DME) and 1-butene were reacted over an MTT-type and a TON-type zeolite, respectively. A sample of zeolite powder was pressed into tablets and the tablets were broken into pieces and sieved. For catalytic testing, the sieve fraction of 40-60 mesh has been used. Prior to reaction, the fresh catalyst in its ammonium-form was treated ex-situ in air at 600 °C for 2 hours.

The reaction was performed using a quartz reactor tube of 3.6 mm internal diameter. The catalyst was heated in argon to the reaction temperature of 525 °C, and a mixture consisting of 3 vol% dimethyl ether, 3 vol% 1-butene, 2 vol% steam balanced in N2 was passed over the catalyst at atmospheric pressure (1 bar). Gas hourly space velocity is based on total gas flow per mass of catalyst and hour (ml/(g_{cat}.h)). Periodically, the effluent from the reactor was analyzed by gas chromatography (GC) to determine the product composition. The composition has been calculated on a weight basis of all hydrocarbons analyzed.

**Table 4**

| Catalyst | MTT SAR 46 | TON SAR 98 |
|---|---|---|
| Time on stream (h) | 1 | 1 |
| GHSV (ml/(g_{cat}.h) | 15000 | 30000 |
| DME conversion (%) | 100 | 99.8 |
| Methane (wt%) | 0.41 | 0.32 |
| Ethylene (wt%) | 21.1 | 16.2 |
| Propylene (wt%) | 48.2 | 45.8 |
| C4 total (wt%) | 22.8 | 28.5 |
| C5 total (wt%) | 2.8 | 6.0 |
| C6-C9 total (wt%) | 3.9 | 2.5 |
| C6-C8 aromatics (wt%) | 0.71 | 0.66 |
| C2=/C3= ratio (wt/wt) | 0.44 | 0.35 |
| %C4 saturates of C4 totals | 2.5 | 1.6 |

In Tables 4 and 5, Cn total (n being an integer) figures include all hydrocarbon species having n carbon atoms; and C6-C9 total refers to all hydrocarbons having between 6,7,8,or 9 carbon atoms, excluding C6-C8 aromatics.

These examples demonstrate that both zeolites with one-dimensional 10-membered ring channels show excellent conversion properties of a DME feed with C4= co-feed to lighter olefins. Except for water (not shown) and C4 olefins, very low amounts of by-products are formed. In particular, the amount of aromatics formed is very low. Moreover, only a small quantity of C4 saturates is formed, so that it is possible to recycle a C4 stream as olefinic co-feed according to the invention, without the need for a butane/butene split.

### Comparative Example 5

MFI zeolites ZSM-5 of Silica-to-Alumina ratios SAR = 55 and 280 were used in experiments further identical to that of MTT in Example 4a. The results are shown in Table 5.

**Table 5**

| Catalyst | MFI SAR 55 | MFI SAR 280 |
|---|---|---|
| Time on stream (h) | 1 | 1 |
| GHSV (ml/(g_{cat}.h) | 15000 | 15000 |
| DME conversion (%) | 100 | 100 |
| Methane (wt%) | 1.0 | 0.82 |
| Ethylene (wt%) | 24.3 | 11.9 |
| Propylene (wt%) | 35.3 | 43.5 |
| C4 total (wt%) | 19 | 28.5 |
| C5 total (wt%) | 5.7 | 8.5 |
| C6-C9 total | 3.4 | 3.6 |
| excl. aromatics (wt%) | | |
| C6-C8 aromatics (wt%) | 11.3 | 3.2 |
| C2=/C3= ratio (wt/wt) | 0.69 | 0.27 |
| % C4 saturates of C4 totals | 47 | 6.4 |

Both MFI oxygenate conversion catalysts show lower total yield of lower olefins (ethylene+propylene) than the Examples 4a and 4b. Moreover, much larger quantities of by-products are formed, i.e. much higher amounts of aromatics, and other C5+ hydrocarbon species. The portion of C4 saturates of total C4 is also much higher, in fact drastically higher for the SAR 55 zeolite. An oxygenate conversion catalyst consisting of the ZSM-5 zeolite with mutidimensional channels is therefore unsuitable for the process of the present invention.

### Example 6

Model calculations similar to those of Example 1 have been conducted, but now for a reactor system 201 comprising three sequential isothermal plug flow reactors. The reactor system 201 is depicted in Figure 3, comprising three sequential isothermal reactors 211,212,213 in fluid communication by lines 228 and 230, with a reactor effluent line 251 from the third reactor 213. In the calculations, oxygenate feedstock (DME, MeOH) was fed via lines 234,235,236, and C4 olefin was fed to the first reactor 211 via line 238. Products (ethylene, propylene and water) are withdrawn after separation from C4 olefins in separation system 270, as generally depicted by line 274. C4= and C4 saturates C4,0 are recycled to the first reactor 211.

The catalyst amount assumed in the model was 5,7.5 and 12.5 tonnes for the reactors 211, 212, 213, respectively. 40 wt% of the catalyst was assumed to be zeolite. A uniform temperature of 522 °C and pressure of 1 bar was assumed. In an isothermal model it is not needed to consider the actual flow of catalyst through the system, therefore no catalyst separation and addition is shown.

The composition of various streams indicated by the reference numerals of Figure 3 are given in Table 6.

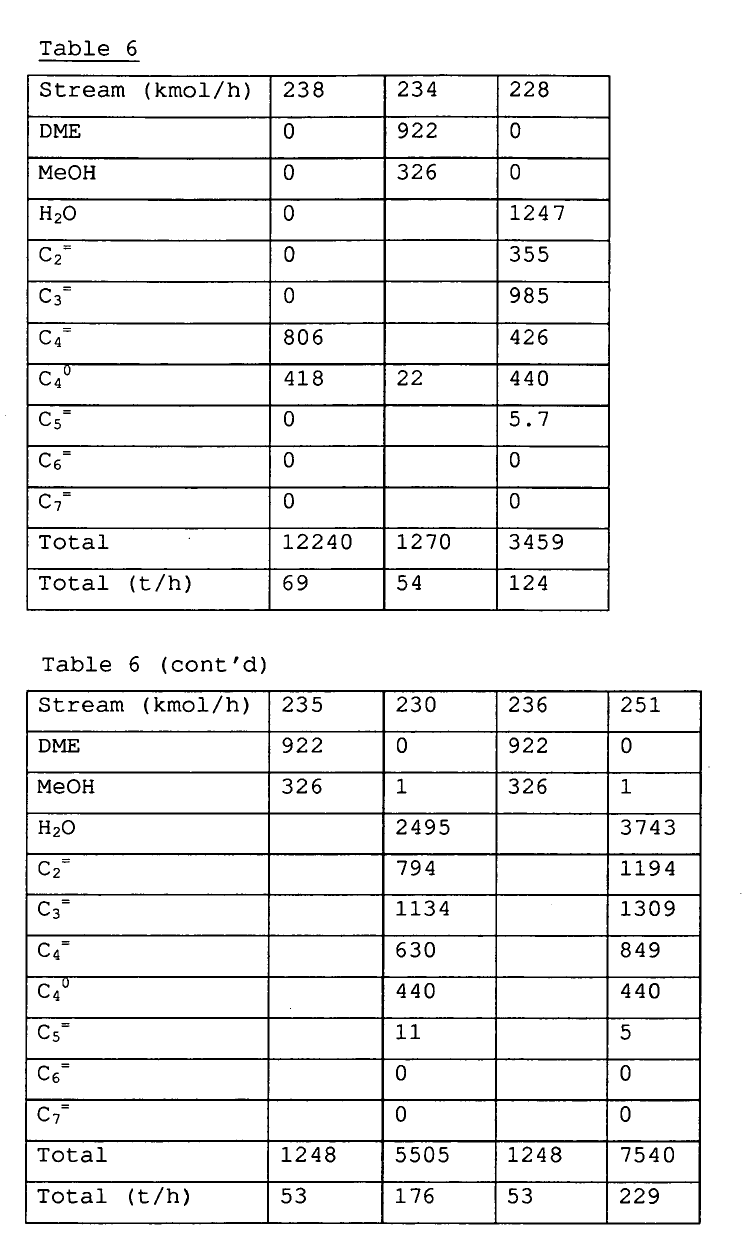

In this process according to the invention, again a very high conversion of oxygenate to light olefins is observed. With the staged addition of oxygenate the selectivity is shifted towards ethylene. The molar ratio of C2=/C3= obtained is 0.91.

## Claims

1. Process for the preparation of an olefinic product, which process comprises the steps of
a) reacting an oxygenate feedstock comprising oxygenate species having an oxygen-bonded methyl group, preferably methanol and/or dimethylether, and an olefinic co-feed, in the presence of an oxygenate conversion catalyst comprising at least 50 wt%, based on total molecular sieve in the oxygenate conversion catalyst, of a molecular sieve having one-dimensional 10-membered ring channels, to prepare an olefinic reaction effluent, wherein the olefinic co-feed comprises less than 10 wt% of C5+ hydrocarbon species;
b) fractionating the olefinic reaction effluent to obtain at least a light olefinic fraction comprising ethylene, and a heavier olefinic fraction comprising C4 olefins and less than 10 wt% of C5+ hydrocarbon species;
c) recycling at least part of the heavier olefinic fraction obtained in step b) as recycle stream to step a), to form at least part of the olefinic co-feed; and
d) withdrawing at least part of the light olefinic fraction as olefinic product.

2. Process according to claim 1, wherein at least 70 wt% of the olefinic co-feed in step a), during normal operation, is formed by the recycle stream of step c), preferably at least 90 wt%, more preferably at least 99 wt%, and most preferably the olefinic co-feed is during normal operation formed by the recycle stream.

3. Process according to claim 1 or 2 wherein the heavier olefinic fraction comprises at least 50 wt% of C4 olefins, and at least a total of 70 wt% of C4 hydrocarbon species.

4. Process according to any one of claims 1-3, wherein the heavier olefinic fraction further comprises propylene.

5. Process according to any one of claims 1-4, wherein the olefinic reaction effluent comprises 10 wt% or less, preferably 5 wt% or less, more preferably 1 wt% or less, of C6-C8 aromatics, based on total hydrocarbons in the effluent.

6. Process according to any one of claims 1-5, wherein the olefinic co-feed comprises less than 5 wt% of C5+ olefins, preferably less than 2 wt% of C5+ olefins, based on total hydrocarbons in the olefinic co-feed.

7. Process according to any one of claims 1-6, wherein step a) is conducted at a temperature of more than 450 °C, preferably at a temperature of 460 °C or higher, more preferably at a temperature of 480 °C or higher.

8. Process according to any one of claims 1-7, wherein the one-dimensional molecular sieve having 10-membered ring channels comprises at least one of a molecular sieve of the MTT-type and/or of the TON-type.

9. Process according to any one of claims 1-8, wherein the oxygenate conversion catalyst comprises more than 50 wt%, preferably at least 65 wt%, based on total molecular sieve in the oxygenate conversion catalyst, of the one-dimensional molecular sieve having 10-membered ring channels.

10. Process according to any one of claims 1-9, wherein the oxygenate conversion catalyst comprises at least 1 wt%, based on total molecular sieve in the oxygenate conversion catalyst, of a further molecular sieve having more-dimensional channels, preferably at least 5 wt%, more preferably at least 8 wt%.

11. Process according to claim 10, wherein the catalyst composition comprises less than 35 wt% of the further molecular sieve, based on the total weight of molecular sieves in the catalyst composition, preferably less than 20 wt%, more preferably less than 18 wt%, still more preferably less than 15 wt%.

12. Process according to claim 10 of 112, wherein the further molecular sieve is a MFI-type molecular sieve, in particular zeolite ZSM-5.

13. Process according to any one of claims 1-12, wherein step a) is performed in a reactor system comprising a riser reactor, preferably a reactor system comprising two or more serially arranged riser reactor stages to obtain a riser reactor effluent from each stage, wherein each riser reactor stage comprises a single riser reactor or a plurality of parallel riser reactors, such that at least part of the riser reactor effluent of a preceding riser reactor stage is fed into a subsequent riser reactor stage

14. Process according to any one of claims 1-13, wherein step a) is performed in a reactor system comprising a plurality of sequential reaction zones, and wherein oxygenate is added to at least two of the sequential reaction zones.

15. Process according to any one of claims 1-14, wherein the oxygenate is obtained as a reaction product of synthesis gas, in particular from synthesis gas generated from fossil fuels, such as from natural gas or oil, or from the gasification of coal.

## Patentansprüche

1. Verfahren zur Herstellung eines olefinischen Produkts, welches Verfahren die Schritte
a) Umsetzen eines Oxygenat-Einsatzmaterials, umfassend Oxygenatspezies mit einer Sauerstoff-gebundenen Methylgruppe, vorzugsweise Methanol und/oder Dimethylether, mit einem olefinischen Co-Einsatzmaterial in Gegenwart eines Oxygenat-Umwandlungskatalysators, umfassend wenigstens 50 Gew.-%, bezogen auf das Gesamtmolekularsieb im Oxygenat-Umwandlungskatalysator, von einem Molekularsieb mit eindimensionalen 10-gliedrigen Ringkanälen, um einen olefinischen Reaktionsabstrom herzustellen, wobei das olefinische Co-Einsatzmaterial wenigstens 10 Gew.-% von C5+-Kohlenwasserstoffspezien umfasst;
b) Fraktionieren des olefinischen Reaktionsabstromes, um wenigstens eine leichte Olefinfraktion, umfassend Ethylen, und eine schwerere Olefinfraktion, umfassend C4-Olefine und weniger als 10 Gew.-% an C5+-Kohlenwasserstoffspezien, zu erhalten;
c) Recyclieren von wenigstens einem Teil der schwereren Olefinfraktion, die im Schritt b) erhalten wird, als Recyclierungsstrom in den Schritt a), um wenigstens einen Teil des olefinischen Co-Einsatzmaterials zu bilden; und
d) Abziehen von wenigstens einem Teil der leichten Olefinfraktion als Olefinprodukt,
umfasst.

2. Verfahren nach Anspruch 1, wobei wenigstens 70 Gew.-% des olefinischen Co-Einsatzmaterials im Schritt a) während des Normalbetriebs vom Recyclierungsstrom von Schritt c) gebildet werden, vorzugsweise wenigstens 90 Gew.-%, stärker bevorzugt wenigstens 99 Gew.-% und am stärksten bevorzugt das olefinische Co-Einsatzmaterial während des Normalbetriebs vom recyclierten Strom gebildet werden.

3. Verfahren nach Anspruch 1 oder 2, wobei die schwerere Olefinfraktion wenigstens 50 Gew.-% an C4-Olefinen und wenigstens insgesamt 70 Gew.-% an C4-Kohlenwasserstoff-spezien umfasst.

4. Verfahren nach einem der Ansprüche 1 bis 3, wobei die schwerere Olefinfraktion ferner Propylen umfasst.

5. Verfahren nach einem der Ansprüche 1 bis 4, wobei der olefinische Reaktionsabstrom 10 Gew.-% oder weniger, vorzugsweise 5 Gew.-% oder weniger, stärker bevorzugt 1 Gew.-% oder weniger an C6-C8-Aromaten, bezogen auf die Gesamtkohlenwasserstoffe im Abstrom, umfasst.

6. Verfahren nach einem der Ansprüche 1 bis 5, wobei das olefinische Co-Einsatzmaterial weniger als 5 Gew.-% an C5+-Olefinen, vorzugsweise weniger als 2 Gew.-% an C5+-Olefinen, bezogen auf die Gesamtkohlenwasserstoffe im olefinischen Co-Einsatzmaterial, umfasst.

7. Verfahren nach einem der Ansprüche 1 bis 6, wobei der Schritt a) bei einer Temperatur von mehr als 450°C, vorzugsweise bei einer Temperatur von 460°C oder darüber, stärker bevorzugt bei einer Temperatur von 480°C oder darüber, durchgeführt wird.

8. Verfahren nach einem der Ansprüche 1 bis 7, wobei das eindimensionale Molekularsieb mit 10-gliedrigen Ringkanälen wenigstens ein Molekularsieb vom MTT-Typ und/oder vom TON-Typ umfasst.

9. Verfahren nach einem der Ansprüche 1 bis 8, wobei der Oxygenat-Umwandlungskatalysator mehr als 50 Gew.-%, vorzugsweise wenigstens 65 Gew.-%, bezogen auf das Gesamtmolekularsieb im Oxygenat-Umwandlungskatalysator, von dem eindimensionalen Molekularsieb mit 10-gliedrigen Ringkanälen umfasst.

10. Verfahren nach einem der Ansprüche 1 bis 9, wobei der Oxygenat-Umwandlungskatalysator wenigstens 1 Gew.-%, bezogen auf das gesamte Molekularsieb im Oxygenat-Umwandlungskatalysator, von einem weiteren Molekularsieb mit mehrdimensionalen Kanälen, vorzugsweise wenigstens 5 Gew.-%, stärker bevorzugt wenigstens 8 Gew.-%, umfasst.

11. Verfahren nach Anspruch 10, wobei die Katalysatorzusammensetzung weniger als 35 Gew.-% des weiteren Molekularsiebs, bezogen auf das Gesamtgewicht der Molekularsiebe in der Katalysatorzusammensetzung, vorzugsweise weniger als 20 Gew.-%, stärker bevorzugt weniger als 18 Gew.-%, noch stärker bevorzugt weniger als 15 Gew.-%, umfasst.

12. Verfahren nach einem der Ansprüche 10 bis 11, wobei das weitere Molekularsieb ein Molekularsieb vom MFI-Typ ist, insbesondere Zeolith ZSM-5.

13. Verfahren nach einem der Ansprüche 1 bis 12, wobei der Schritt a) in einem Reaktorsystem, umfassend einen Riser-Reaktor, vorzugsweise einem Reaktorsystem, umfassend zwei oder mehr seriell angeordnete Riser-Reaktor-Stufen, um einen Riser-Reaktor-Abstrom aus jeder Stufe zu erhalten, durchgeführt wird, wobei jede Riser-Reaktor-Stufe einen einzelnen Riser-Reaktor oder eine Mehrzahl von parallelen Riser-Reaktoren umfasst, sodass wenigstens ein Teil des Riser-Reaktor-Abstroms aus einer vorhergehenden Riser-Reaktor-Stufe in die darauf folgende Riser-Reaktor-Stufe zugeführt wird.

14. Verfahren nach einem der Ansprüche 1 bis 13, wobei der Schritt a) in einem Reaktorsystem, umfassend eine Mehrzahl von sequentiellen Reaktionszonen, durchgeführt wird, und wobei das Oxygenat zu wenigstens zwei der sequentiellen Reaktionszonen zugesetzt wird.

15. Verfahren nach einem der Ansprüche 1 bis 14, wobei das Oxygenat als ein Reaktionsprodukt von Synthesegas, insbesondere von Synthesegas, welches aus fossilen Brennstoffen gebildet wird, wie aus Erdgas oder -öl, oder aus der Vergasung von Kohle, erhalten wird.

## Revendications

1. Procédé de préparation d'un produit oléfinique, ledit procédé comprenant les étapes suivantes :
a) la réaction d'une charge d'alimentation de composés oxygénés comprenant des espèces oxygénées contenant un groupe méthyle lié à un oxygène, de préférence le méthanol et/ou le diméthyléther, et d'une co-charge oléfinique, en présence d'un catalyseur de conversion de composés oxygénés comprenant au moins 50 % en poids, sur la base du tamis moléculaire total dans le catalyseur de conversion de composés oxygénés, d'un tamis moléculaire contenant des canaux monodimensionnels à cycle à 10 chaînons, pour préparer un effluent réactionnel oléfinique, la co-charge oléfinique comprenant moins de 10 % en poids d'espèces hydrocarbonées en C₅₊ ;
b) le fractionnement de l'effluent réactionnel oléfinique pour obtenir au moins une fraction oléfinique légère comprenant de l'éthylène, et une fraction oléfinique plus lourde comprenant des oléfines en C₄ et moins de 10 % en poids d'espèces hydrocarbonées en C₅₊ ;
c) le recyclage d'au moins une partie de la fraction oléfinique plus lourde obtenue dans l'étape b) en tant que courant de recyclage vers l'étape a), pour former au moins une partie de la co-charge oléfinique ; et
d) le retrait d'au moins une partie de la fraction oléfinique légère en tant que produit oléfinique.

2. Procédé selon la revendication 1, dans lequel au moins 70 % en poids de la co-charge oléfinique dans l'étape a), lors d'un fonctionnement normal, sont formés par le courant de recyclage de l'étape c), de préférence au moins 90 % en poids, et de manière davantage préférée au moins 99 % en poids, et de manière préférée entre toutes la co-charge oléfinique est formée, lors d'un fonctionnement normal, par le courant de recyclage.

3. Procédé selon la revendication 1 ou 2, dans lequel la fraction oléfinique plus lourde comprend au moins 50 % en poids d'oléfines en C₄ et au moins 70 % en poids au total d'espèces hydrocarbonées en C₄.

4. Procédé selon l'une quelconque des revendications 1 à 3, dans lequel la fraction oléfinique plus lourde comprend en outre du propylène.

5. Procédé selon l'une quelconque des revendications 1 à 4, dans lequel l'effluent réactionnel oléfinique comprend 10 % en poids ou moins, de préférence 5 % en poids ou moins, et de manière davantage préférée 1 % en poids ou moins, de composés aromatiques en C₆ à C₈, sur la base des hydrocarbures totaux dans l'effluent.

6. Procédé selon l'une quelconque des revendications 1 à 5, dans lequel la co-charge oléfinique comprend moins de 5 % en poids d'oléfines en C₅₊, de préférence moins de 2 % en poids d'oléfines en C₅₊, sur la base des hydrocarbures totaux dans la co-charge oléfinique.

7. Procédé selon l'une quelconque des revendications 1 à 6, dans lequel l'étape a) est menée à une température supérieure à 450 °C, de préférence à une température supérieure ou égale à 460 °C, et de manière davantage préférée à une température supérieure ou égale à 480 °C.

8. Procédé selon l'une quelconque des revendications 1 à 7, dans lequel le tamis moléculaire monodimensionnel contenant des canaux à cycle à 10 chaînons comprend au moins un tamis moléculaire de type MTT et/ou de type TON.

9. Procédé selon l'une quelconque des revendications 1 à 8, dans lequel le catalyseur de conversion de composés oxygénés comprend plus de 50 % en poids, de préférence au moins 65 % en poids, sur la base du tamis moléculaire total dans le catalyseur de conversion de composés oxygénés, du tamis moléculaire monodimensionnel contenant des canaux à cycle à 10 chaînons.

10. Procédé selon l'une quelconque des revendications 1 à 9, dans lequel le catalyseur de conversion de composés oxygénés comprend au moins 1 % en poids, sur la base du tamis moléculaire total dans le catalyseur de conversion de composés oxygénés, d'un tamis moléculaire supplémentaire contenant des canaux multidimensionnels, de préférence au moins 5 % en poids, et de manière davantage préférée au moins 8 % en poids.

11. Procédé selon la revendication 10, dans lequel la composition catalytique comprend moins de 35 % en poids du tamis moléculaire supplémentaire, sur la base du poids total des tamis moléculaires dans la composition catalytique, de préférence moins de 20 % en poids, et de manière davantage préférée moins de 18 % en poids, ou mieux moins de 15 % en poids.

12. Procédé selon la revendication 10 et 11, dans lequel le tamis moléculaire supplémentaire est un tamis moléculaire de type MFI, en particulier la zéolithe ZSM-5.

13. Procédé selon l'une quelconque des revendications 1 à 12, dans lequel l'étape a) est effectuée dans un système de réacteurs comprenant un réacteur à colonne montante, de préférence un système de réacteurs comprenant deux ou plus de deux étages de réacteur à colonne montante disposés en série pour obtenir un effluent de réacteur à colonne montante provenant de chaque étage, dans lequel chaque étage de réacteur à colonne montante comprend un seul réacteur à colonne montante ou une pluralité de réacteurs à colonne montante en parallèle, de manière à ce qu'au moins une partie de l'effluent de réacteur à colonne montante d'un étage précédent de réacteur à colonne montante soit introduit dans un étage suivant de réacteur à colonne montante.

14. Procédé selon l'une quelconque des revendications 1 à 13, dans lequel l'étape a) est effectuée dans un système de réacteurs comprenant une pluralité de zones réactionnelles séquentielles, et dans lequel un composé oxygéné est ajouté dans au moins deux des zones réactionnelles séquentielles.

15. Procédé selon l'une quelconque des revendications 1 à 14, dans lequel le composé oxygéné est obtenu sous la forme d'un produit réactionnel de gaz de synthèse, en particulier à partir d'un gaz de synthèse généré à partir de carburants fossiles, tels que le gaz naturel ou le pétrole, ou à partir de la gazéification du charbon.
